Europäisches Patentamt

European Patent Office

Office européen des brevets

(19)

(11) Veröffentlichungsnummer : **0 194 425**
**B1**

(12) **EUROPÄISCHE PATENTSCHRIFT**

(45) Veröffentlichungstag der Patentschrift :
30.09.87

(21) Anmeldenummer : 86100965.2

(22) Anmeldetag : 24.01.86

(51) Int. Cl.⁴ : **C 07 C 11/04**, C 07 C 9/06,
C 07 C 2/84// C07C9/04

(54) Verfahren zur Herstellung von Ethylen-Ethan-Gemischen.

(30) Priorität : 04.02.85 DE 3503664

(43) Veröffentlichungstag der Anmeldung :
17.09.86 Patentblatt 86/38

(45) Bekanntmachung des Hinweises auf die Patenterteilung : 30.09.87 Patentblatt 87/40

(84) Benannte Vertragsstaaten :
AT BE CH DE FR GB IT LI NL

(56) Entgegenhaltungen :
DE-A- 3 237 079

(73) Patentinhaber : Akzo GmbH
Postfach 10 01 49 Kasinostrasse 19-23
D-5600 Wuppertal-1 (DE)

(72) Erfinder : Wohlfahrt, Klaus, Dr., Dipl.-Ing.
Römerstrasse 88
D-8753 Obernburg (DE)
Erfinder : Bergfeld, Manfred, Dr., Dipl.-Chem.
August-Pfeffer-Strasse 6
D-8765 Erlenbach-Mechenhard (DE)
Erfinder : Zengel, Hans, Dr., Dipl.-Ing.
Nordring 6
D-8751 Kleinwallstadt (DE)

EP 0 194 425 B1

**Beschreibung**

Die vorliegende Erfindung betrifft ein Verfahren zur Herstellung von Ethylen und Ethan durch oxidative Kopplung von Methan mittels Sauerstoff oder eines sauerstoffhaltigen Gases in Gegenwart eines Katalysators.

Die Herstellungsverfahren für das in der chemischen Synthese vielfältig eingesetzte Ausgangsmaterial Ethylen beruhen zur Zeit fast ausschließlich auf dem Kracken von Erdöldestillaten oder Erdgaskondensaten aus « nassem » Erdgas (Ethan und höhere Kohlenwasserstoffe). Dem technischen Krackprozeß müssen noch umfangreiche Reinigungsschritte und Gastrennverfahren nachgeschaltet werden, um das Ethylen in der für die Weiterverarbeitung erforderlichen Reinheit zu erhalten. Weniger aufwendige Aufarbeitungsschritte sind bei der Herstellung von Ethylen aus Ethan erforderlich ; doch ist die Verfügbarkeit von Ethan begrenzt.

Demgegenüber ist Methan ein in den natürlichen Lagerstätten reichlich vorhandener Rohstoff. So enthält Erdgas das Methan zu über 90 %. Es besteht daher ein Interesse an einem wirtschaftlichen Verfahren zur Herstellung von Ethylen aus Methan durch oxidative Kopplung.

Über die Herstellung von niederen Olefinen aus Methan ist vielfach berichtet worden. So beschreiben T. Inui et al. in Hydrocarbon Proc., Nov. 1982, Seite 117 ein Verfahren, bei dem ein Gemisch niederer Olefine in drei Reaktionsstufen über Synthesegas und Methanol erhalten wird. Y.C. Hu beschreibt in Hydrocarbon Proc., Mai 1983, Seite 88 ein 2-Stufen-Verfahren, bei dem das in der ersten Stufe gebildete Synthesegas durch Fischer-Tropsch-Reaktion in ein Gemisch aus Olefinen, Paraffinen und Kohlendioxid umgesetzt wird. Diese Verfahren haben insbesondere den Nachteil, daß unter Druck gearbeitet werden muß, Koksbildung festzustellen ist und mehrstufige Synthesen erforderlich sind.

Es ist aber auch schon die Umsetzung von Methan in Gegenwart von Sauerstoff bei Atmosphärendruck in einem Reaktionsschritt durchgeführt worden. So berichten G.E. Keller und M.M. Bhasin in J. Catal. 73 (1982), Seiten 9-19 von einem Verfahren, bei dem die Umsetzung in Gegenwart zahlreicher Metalloxide als Katalysator bei 500 bis 1 000 °C untersucht wurde und als Reaktionsprodukte neben Kohlenmonoxid und Kohlendioxid ein Gemisch von Ethylen und Ethan gebildet wurde. Diesem Verfahren haftet jedoch der Mangel einer sehr niedrigen $C_2$-Kohlenwasserstoff-Selektivität an. Zur Verbesserung der Selektivitäten schlagen diese Autoren eine allerdings apparativ sehr aufwendige und komplizierte Arbeitsweise vor.

Unter Hinweis auf die Mängel dieser Vorveröffentlichung wird nun in der DE-OS 3 237 079 ein Verfahren vorgeschlagen, das auch ohne aufwendige und komplizierte Prozeßführung zu vergleichbaren, teilweise sogar auch besseren Selektivitäten und höheren Raumzeitausbeuten an $C_2$-Kohlenwasserstoffen führt (vgl. DE-OS 3 237 079, Seite 2, letzter Absatz, bis Seite 3, Absatz 3). Gemäß dieser Druckschrift wird ein Verfahren zur Erzeugung von Ethan und/oder Ethylen beschrieben, bei dem Methan und Sauerstoff in Gegenwart eines in einer Wirbelschicht fluidisierten oder in einem Reaktor fest angeordneten Katalysators bei Temperaturen zwischen 500 und 900 °C in einem bestimmten Bereich der Partialdruck-Verhältnisse von Methan und Sauerstoff umgesetzt werden. Der Katalysator soll ein Oxid mehrwertiger Metalle sein (vgl. DE-OS 3 237 079, Seite 3, letzter Absatz). Als bevorzugte Katalysatoren werden Produkte mit Oxiden des Bleis, Antimons, Zinns, Wismuts, Cadmiums, Thalliums und Indiums oder deren Mischungen als aktive Komponente genannt, als besonders bevorzugt jedoch Bleioxid oder seine Mischung mit Antimonoxid. Die Metalloxide können als solche oder fein verteilt auf der Oberfläche eines Trägermaterials wie Aluminiumoxid oder Siliciumdioxid zum Einsatz kommen (vgl. Anspruch 6 sowie Seite 4, Absatz 4). Wie die Ergebnisse der Ausführungsbeispiele in Tabelle 1 nun zeigen, wird jedoch auch mit dem besonders bevorzugten Bleioxid (auf einem Träger) eine Selektivität für Kohlenwasserstoffe von maximal nur ca. 52,9 % (Methan-Umsatz 7 %) erreicht. — Über weitergehende Arbeiten zur Erhöhung der Selektivität haben W. Hinsen, W. Bytyn und M. Baerns auf dem 8. Internationalen Kongreß der Katalyse 1984 in Berlin berichtet. Gemäß Kongreß-Buch « 8th International Congress on Catalysis », Vol. III, Seiten 581-592, Verlag Chemie, Weinheim 1984 kann die Kohlenwasserstoff-Selektivität des Bleioxid-Katalysators bei geeigneter Auswahl des Trägermaterials und durch Alkalizusatz erhöht werden. Von den untersuchten Trägermaterialien α-Aluminiumoxid, γ-Aluminimoxid, Titandioxid, Aluminiumsilikat, Silikagel erwies sich dabei γ-Aluminiumoxid am geeignetsten ; es führte zu Selektivitäten von maximal 57,7 % (Methan-Umsatz 7,1 %). Für eine kommerzielle Nutzung des Verfahrens der oxidativen Kopplung von Methan besteht jedoch das Bedürfnis nach einer wesentlichen Erhöhung der Selektivität und des Methan-Umsatzes.

Aufgabe der vorliegenden Erfindung ist es daher, Methan nach dem im grundlegenden Prinzip bekannten Verfahren der oxidativen Kopplung in einer katalysierten Reaktion mit höherer Selektivität bzw. brauchbaren Methan-Umsätzen insbesondere zu $C_2$-Kohlenwasserstoffen umzusetzen.

Diese Aufgabe wird gelöst durch ein Verfahren zur Herstellung von Ethan und Ethylen durch Oxidation von Methan mittels Sauerstoff oder eines sauerstoffhaltigen Gases in Gegenwart einer Metallverbindung als Katalysator bei einer Reaktionstemperatur von 600 bis 1 000 °C, das dadurch gekennzeichnet ist, daß man als Katalysator Chloride, Bromide und Jodide der Alkali- und Erdalkalielemente, der Elemente der Nebengruppe IIIb des Periodensystems, der Elemente mit der Ordnungszahl 24 bis 30, der Lanthinidenelemente, des Silbers, Cadmiums, Bleis und Wismuts einzeln oder im Gemisch

2

einsetzt, wobei das Atomgewicht der Alkali-, Erdalkali- und IIIb-Nebengruppen-Elemente nicht größer als 139 ist.

Es war überraschend, daß mit Katalysatoren aus der Gruppe der Metallhalogenide allein oder im Gemisch wesentlich höhere Selektivitäten und höhere Methan-Umsätze resultieren, als bisher gemäß Vorveröffentlichungen ermittelt wurden.

Im allgemeinen werden verbesserte Selektivitäten bereits allein mit einem der Metallchloride, -bromide oder -jodide erzielt, jedoch ist in diesen Fällen vielfach erniedrigter.

Umsatz festzustellen. Die Katalysatoren werden daher bevorzugt auf einem Träger eingesetzt. Vorteilhaft zur Erreichung brauchbarer Umsätze ist, daß der Halogenid-Katalysator bei Betriebstemperatur geschmolzen vorliegt. Beispielsweise liegt Calciumchlorid bei einer Betriebstemperatur von 750 °C in festem Zustand vor ; eine Erhöhung der Temperatur auf 800 °C läßt das Calciumchlorid schmelzen und den Methan-Umsatz von 5,8 % auf 14,2 % ansteigen (Katalysator : 0,18 mol $CaCl_2$ auf 20 g Bims). Es empfiehlt sich also, die Betriebstemperatur so zu wählen, daß sie leicht oberhalb des Katalysator-Schmelzpunktes liegt. Um zu hohe Betriebstemperaturen zu vermeiden, setzt man den jeweiligen Halogenid-Katalysator in Kombination mit mindestens einer Sekundärkomponente zur Schmelzpunkterniedrigung ein. Im allgemeinen ist die Sekundärkomponente ebenfalls ein Halogenid der Alkali- und Erdalkalimetalle, der Elemente der Nebengruppe IIIb des Periodensytems, der Elemente mit der Ordnungszahl 24 bis 30, der Lanthanidenelemente, des Silbers, Cadmiums, Bleis und Wismuts.

Selbstverständlich sind unter Chloriden, Bromiden und Jodiden gemäß Anspruch 1 auch die Oxichloride, Oxibromide und Oxijodide von denjenigen der in Anspruch 1 genannten Elemente zu verstehen, welche derartige Oxihalogenide zu bilden vermögen, wie zum Beispiel Wismut.

Bevorzugt eingesetzte Chloride, Bromide und Jodide von Elementen mit der Ordnungszahl 24 bis 30 als Katalysator sind diejenigen der Elemente Chrom, Mangan, Eisen, Kupfer und Zink.

Es hat sich ferner als vorteilhaft erwiesen, als Katalysator auch Halogenide von Lanthanidenelement-Gemischen einzusetzen, wie sie in natürlich vorkommenden Seltenerdmineralien zusammengesetzt sind. Von den natürlich vorkommenden Seltenerdmineralien sind Ceriterden oder Yttererden, zum Beispiel Monazit, zur Herstellung erfindungsgemäßer Katalysatoren geeignet. Ceriterden bestehen aus Gemischen von Oxiden des Lanthans, Cers, Praseodyms, Neodyms, Samariums und Europiums, während Ytterden aus Gemischen von Oxiden des Gadoliniums, Terbiums, Dysprosiums, Holmiums, Erbiums, Ytterbiums, Thuliums, Lutetiums, Ytteriums und Thoriums bestehen, wobei die jeweilige Zusammensetzung je nach Mineral schwankt. Zusammensetzungen verschiedener Ceriterden- und Yttererden-Mineralien und speziell von Monazit sind zum Beispiel aus Ullmanns Encyclopädie der technischen Chemie, Verlag Chemie, Weinheim 1982, Band 21, Seite 238, Tabelle 4 ersichtlich.

Im allgemeinen erhält man eine brauchbare Halogenid-Katalysatorkombination, wenn man als Primärkomponente ein billiges, meist hochschmelzendes Alkali- oder Erdalkali-halogenid oder deren Gemische und als Sekundärkomponente ein Halogenid der Schwermetalle oder Gemische davon wählt. Die Schwermetallhalogenide einschließlich der Lanthanidenhalogenide haben vielfach zusätzlich eine Promotorwirkung im Sinne einer Ethylen-Ausbeuteerhöhung.

Das erfindungsgemäße Verfahren kann bei Temperaturen im Bereich zwischen 600 und 1 000 °C durchgeführt werden. Bevorzugt wird ein Temperaturbereich zwischen 650 und 850 °C.

Die Chlorid-, Bromid- und Jodid-Katalysatoren werden bevorzugt auf einem Träger eingesetzt, welcher aus Bimsstein, Kieselgel, Kieselgur, gefällter Kieselsäure und/oder Oxiden der Erdalkalielemente und/oder Aluminiumoxid, Siliciumdioxid, Zinkoxid, Titandioxid, Zirkondioxid und/oder Siliciumcarbid besteht. Von den als Träger eingesetzten Oxiden der Erdalkalielemente werden Magnesium- und Calciumoxid bevorzugt.

Die Katalysatoren werden vorteilhaft in einer Menge von 0,004 bis 0,300 Mol Katalysator auf 100 ml Träger eingesetzt. Besonders vorteilhaft sind 0,012 bis 0,190 Mol Katalysator auf 100 ml Träger.

Sämtliche Katalysatoren können sowohl im Fließbett als auch im Festbett angeordnet werden. Die geschmolzenen Halogenide können auch als Flüssigkeit von dem Reaktionsgas blasenförmig durchströmt werden.

Die Katalysatoren können wie folgt hergestellt werden :

Für den Einsatz der Halogenid-Katalysatoren auf einem Träger werden die Aktivkomponenten in Wasser oder einem anderen geeigneten Lösungsmittel gelöst und das Trägermaterial in Pelletform in der resultierenden Lösung suspendiert.

Die Lösung mit suspendiertem Träger wird im Vakuum zur Trockene eingeengt und bei ca. 100 °C im Vakuum getrocknet.

Bei Einsatz ohne Träger kann der Katalysator als solcher verwendet werden, daß heißt ohne weitere Zusatzmittel. Es ist aber auch möglich, ein Bindemittel mitzuverwenden. Die Herstellung eines Katalysators mit Bindemittel wird im folgenden beschrieben.

Die jeweiligen Komponenten werden in einer Reibschale mit Natronwasserglas angeteigt und gut durchgeknetet. Anschließend wird etwas konzentrierte Ammoniaklösung zugefügt und durchgeknetet, um die Polymerisation des Wasserglases zum Katalysator-Bindemittel einzuleiten, wobei die Masse langsam erstarrt. Mit dieser Masse wird eine Metallplatte beschichtet (Schichthöhe ca. 5 mm). Nach dem Trocknen dieser Schicht im Vakuum bei ca. 80 °C werden die hierbei entstandenen spröden Halogenid-Katalysator-Partikel auf die gewünschte Korngröße zerkleinert.

Die nachstehend beschriebenen Versuche wurden in einer Quarzröhre mit einem inneren Durchmesser von 11,3 mm durchgeführt. Nach Einfüllen des Katalysators in diese Röhre wird dieser im Stickstoffstrom auf Reaktionstemperatur gebracht. Die Röhre wird auf einer Länge von 0,5 m mittels eines Strahlungsrohrofens erhitzt. Methan und Sauerstoff oder Luft werden über Strömungsmesser in diesen Reaktor geführt und über den jeweiligen Katalysator geleitet. Das austretende Reaktionsgemisch wird auf einer Temperatur oberhalb 80 °C gehalten und direkt zur Bestimmung im Gaschromatographen geleitet. — Dieses Gemisch enthält als Folgeprodukte des Oxidationsvorgangs Kohlenmonoxid, Kohlenoxid, Wasser, Ethylen, Ethan und in geringeren Mengen höhere Kohlenwasserstoffe, insbesondere $C_3$- und $C_4$-Kohlenwasserstoffe. Rußbildung ist nicht festzustellen. Im austretenden Gasgemisch sind auch Halogenwasserstoff und Halogen festzustellen. Ihr Anteil im Gasgemisch ist allerdings sehr gering und hat daher praktisch keinen Nachteil für die Weiterverarbeitung der Verfahrensprodukte.

Es ist damit also auch eine gewisse Desaktivierung des Katalysators verbunden, die jedoch durch eine Dosierung von Halogenwasserstoff in der Zuleitung oder durch eine ständige Regenerierung des Katalysators (Fließbett) leicht ausgeglichen werden kann. Wenn dies erforderlich wird, wird bevorzugt so gearbeitet, daß man gleichzeitig mit dem Methan und dem Sauerstoff oder dem sauerstoffhaltigen Gas den dem jeweils verwendeten Halogenidkatalysator entsprechenden Halogenwasserstoff kontinuierlich oder intermittierend über den Katalysator leitet. Ein Überschuß Halogenwasserstoff schadet nicht. Die Abtrennung dieses Überschusses aus dem austretenden Gasgemisch erübrigt sich sogar, wenn das erhaltene Ethylen in üblichen Verfahren weiterverarbeitet wird, in denen Halogenwasserstoff zugesetzt werden muß. In diesem Falle würde man den Halogenwasserstoff geeigneterweise in einer derartigen Rate zuleiten, daß im resultierenden Gasgemisch Halogenwasserstoff und Ethylen in dem für ein solches übliches Verfahren erforderlichen Mengenverhältnis vorliegen.

Eine derartige Arbeitsweise ist insbesondere für eine nachgeschaltete Oxichlorierung von Ethylen mittels Chlorwasserstoff hervorragend geeignet, wenn die vorangehende oxidative Kopplung des Methans mit einem Chlorid-Katalysator durchgeführt wird.

Sollte dennoch eine Halogenwasserstoff-Trennoperation erforderlich sein, so fällt der Halogenwasserstoff in Form von stark verdünnter Halogenwasserstoffsäure bei der Kondensation des Produktwassers an. — Im Falle einer nachzuschaltenden Vinylchlorid-Herstellung könnte die dann vorliegende stark verdünnte Salzsäure mit dem nach der Oxichlorierung anfallenden Kondensat von Produktwasser und nichtumgesetztem Chlorwasserstoff vereinigt werden.

Die einzelnen in zahlreichen Versuchen gewählten Verfahrensbedingungen und Ergebnisse sind in den Tabellen 1-6 zusammengefaßt.

Von wesentlichem Einfluß auf Selektivität und Umsatz ist das Verhältnis von Methan und Sauerstoff im eingespeisten Gasgemisch, wie dies in Tabelle 1 veranschaulicht ist.

Tabelle 1 : 30 ml eines aus 0,06 Mol Calciumchlorid auf 20 g Bimsstein bestehenden Katalysators, 750 °C.

| Zuleitung (l/h) | | $CH_4$-Umsatz (%) | Selektivität (%) | | | | | |
|---|---|---|---|---|---|---|---|---|
| Methan | Sauerstoff | | $CO+CO_2$ | $C_2H_4$ | $C_2H_6$ | $C_3H_8$ | $C_4H_{10}$ | $\sum KW$ |
| 90 | 10 | 9,4 | 9,7 | 68,6 | 15,4 | 3,5 | 2,8 | 90,3 |
| 80 | 20 | 15,5 | 15,7 | 69,5 | 10,5 | 2,1 | 2,6 | 84,7 |
| 66 | 34 | 19,9 | 26,1 | 60,4 | 9,3 | 1,3 | 2,9 | 73,9 |

Durch eine Erniedrigung des Sauerstoffanteils kann also die Selektivität verbessert werden, während durch Erhöhung des Sauerstoffanteils der Methanumsatz erhöht wird.

Eine andere Möglichkeit zur weiteren Verbesserung der Selektivität bei gleichzeitiger Erhöhung des Methanumsatzes ist eine Verfahrensweise mit Seiteneinspeisung des Sauerstoffs. Hierbei wird am einen Ende der Reaktorröhre nur das Methan zugeführt, während die Gesamtmenge des Sauerstoffs auf mehrere seitlich angebrachte Eintrittsöffnungen verteilt in die Röhre eingespeist wird.

In Tabelle 2 sind die Resultate von Versuchen mit zahlreichen Halogenid-Katalysatoren zusammengestellt.

0 194 425

Tabelle 2 :   66 l Methan/h
66 l Methan/h
33 l Luft (25 °C)/h
750 °C

ca. 50 ml Katalysator (Aktivkomponente + Träger)
0,06 Mol Aktivkomponente auf ca. 60 ml Träger

| Vers. Nr. | Katalysator * (Aktivkomponente) | Träger | Umsatz $CH_4$ % | KW-Selektivität (%) | | | | |
|---|---|---|---|---|---|---|---|---|
| | | | | $C_2H_4$ | $C_2H_6$ | $C_3H_8$ | $C_4H_8$ | $\sum \overline{KW}$ |
| 1 | $CaCl_2$ | kein Träger | 0,9 | 49,1 | 42,1 | 0 | 0 | 91,2 |
| 2 | $CaCl_2$ | Bimsstein | 11,6 | 71,0 | 11,1 | 1,2 | 1,1 | 84,4 |
| 3 | $MgCl_2$ | dito | 5,3 | 38,2 | 49,1 | 5,2 | 0 | 92,5 |
| 4 | LiCl | dito | 5,1 | 44,0 | 45,8 | 4,5 | 0 | 94,3 |
| 5 | CsCl | dito | 4,5 | 30,4 | 54,0 | 3,1 | 0 | 87,5 |
| 6 | $MnCl_2$ | dito | 14,4 | 71,5 | 12,5 | 3,9 | 2,6 | 90,5 |
| 7 | $PbCl_2$ | dito | 10,5 | 47,5 | 18,3 | 5,9 | 6,9 | 78,6 |
| 8 | $CdCl_2$ | dito | 3,7 | 15,8 | 36,0 | 0 | 0 | 51,8 |
| 9 | $FeCl_2$ | dito | 13,9 | 54,6 | 20,1 | 4,9 | 6,5 | 86,1 |
| 10 | SEM-Chlorid ** | dito | 13,8 | 65,8 | 16,5 | 1,9 | 0 | 84,2 |
| 11 | $CaCl_2/MgCl_2$ (2:1) | dito | 14,2 | 50,9 | 24,3 | 7,9 | 8,4 | 91,5 |
| 12 | $CaCl_2$/SEM-Chlorid (2:1) ** | dito | 11,0 | 70,3 | 14,6 | 3,2 | 0 | 88,1 |
| 13 | $CaCl_2/LiCl/MnCl_2$ (1:1:1) | dito | 9,9 | 52,3 | 32,1 | 5,8 | 0 | 90,2 |
| 14 | $CaCl_2/LiCl/PrCl_3$ (1:1:1) | dito | 14,3 | 71,2 | 13,3 | 3,5 | 1,9 | 89,9 |
| 15 | $CaCl_2/LiCl/CeCl_3$ (1:1:1) | dito | 14,2 | 70,2 | 14,1 | 4,1 | 3,2 | 91,6 |

* Zahlenangaben bei Katalysatormischungen = Molverhältnisse

** SEM-Chlorid = Gemisch von Chloriden der Selten-Erdelemente, wie sie aus Monazit (Gemisch von Oxiden von Lanthan, Cer, Praseodym, Neodym, Samarium) erhalten werden.

Fortsetzung
Tabelle 2 :   66 l Methan/h
              33 l Luft (25 °C)/h
              750 °C

ca. 50 ml Katalysator (Aktivkomponente + Träger)
0,06 Mol Aktivkomponente auf ca. 60 ml Träger

| Vers. Nr. | Katalysator * (Aktivkomponente) | Träger | Umsatz $CH_4$ % | KW-Selektivität (%) | | | | |
|---|---|---|---|---|---|---|---|---|
| | | | | $C_2H_4$ | $C_2H_6$ | $C_3H_8$ | $C_4H_8$ | $\sum KW$ |
| 16 | $BaBr_2$ | Bimsstein | 19,0 | 38,1 | 5,8 | 0 | 38,6 | 82,5 |
| 17 | KJ | dito | 3,0 | 22,1 | 30,2 | 0 | 0 | 52,3 |
| 18 | $CaJ_2$ | dito | 8,8 | 18,0 | 17,2 | 0 | 0 | 35,2 |
| 19 | BiOCl | dito | 10,7 | 44,9 | 18,2 | 3,2 | 0 | 66,3 |
| 20 | $CaBr_2$ | Magnesiumoxid | 13,3 | 34,4 | 9,7 | 0,8 | 14,3 | 59,2 |
| 21 | $BaBr_2$ | Siliciumcarbid | 8,9 | 49,8 | 21,4 | 2,9 | 0 | 74,1 |
| 22 | $BaBr_2$ | Calciumoxid | 8,9 | 36,4 | 17,0 | 1,5 | 13,9 | 68,8 |
| 23 | $BaBr_2$ | Wasserglas gebundenes Zinkoxid | 11,8 | 41,0 | 25,6 | 2,9 | 3,9 | 73,4 |
| 24 | KBr | Wasserglas gebundenes Titanoxid | 11,6 | 46,2 | 20,7 | 2,2 | 4,9 | 74,0 |
| 25 | $MnCl_2$ | Kieselgur | 12,5 | 63,0 | 7,2 | 2,1 | 10,5 | 82,8 |
| 26 | $MnCl_2$ | gefällte Kieselsäure | 10,1 | 52,2 | 16,9 | 2,6 | 0 | 71,7 |
| 27 | $LaCl_3$ | kein Träger | 7,6 | 56,2 | 14,1 | 1,8 | 0 | 72,1 |
| 28 | $BaCl_2$ | dito | 0,7 | 31.8 | 50,0 | 0 | 0 | 81,8 |
| 29 | $MnCl_2$ (geschmolzen) | dito | 7,8 | 37,9 | 6,6 | 4,3 | 0 | 48,8 |

0 194 425

Der Tabelle kann leicht entnommen werden, welche Bedingungen für bestimmte Zielsetzungen hinsichtlich der Endprodukt-Zusammensetzung besonders geeignet sind. So kann in einigen Fällen durch entsprechende Wahl der Katalysatoren, wenn gewünscht, der Anteil der Kohlenwasserstoffe mit mehr als zwei Kohlenstoffatomen zurückgedrängt werden. In anderen Fällen kann durch entsprechende Bedingungen (Temperatur, Katalysator) das jeweils gewünschte Verhältnis Ethylen : Ethan eingestellt werden. Im allgemeinen wird ein möglichst hoher Ethylen-Anteil angestrebt sein.

Aus der Tabelle ist vor allem aber ersichtlich, daß in einigen Fällen besonders hohe Kohlenwasserstoff-Selektivitäten von mehr als 90 % bei zum Teil sogar sehr hohen Aktivitäten (Methan-Umsatz maximal 19,0 %) erfindungsgemäß erreicht werden können.

Das erfindungsgemäße Verfahren ist somit den bisher beschriebenen Verfahren überlegen und für eine wirtschaftliche Herstellung von Ethan und Ethylen durch oxidative Kopplung von Methan geeignet.

Hierfür empfiehlt sich eine Prozeßführung mit niedrigem Sauerstoff-Anteil im Reaktor, wie sie zum Beispiel mit einem Fließbettreaktor, Kreislaufreaktor, Seiteneinspeisung oder anderen Maßnahmen der Konzentrationsführung erreicht werden kann.

Bei der Wahl der jeweils geeigneten Verfahrensanlage sollte im Hinblick auf das Material berücksichtigt werden, daß im Reaktionsgemisch korrosiv wirksame Komponenten in Form von Halogen bzw. Halogenwasserstoff anwesend sind.

Das Reaktionsgemisch kann bei technischer Verfahrensweise mittels konventioneller Raffineriegastechnologie getrennt oder gegebenenfalls ohne Trennung zum Beispiel zu halogenierten Produkten, zum Beispiel Vinylchlorid, umgesetzt werden.


**Patentansprüche**

1. Verfahren zur Herstellung von Ethan und Ethylen durch Oxidation von Methan mittels Sauerstoff oder eines sauerstoffhaltigen Gases in Gegenwart einer Metallverbindung als Katalysator bei einer Reaktionstemperatur von 600 bis 1 000 °C, dadurch gekennzeichnet, daß man als Katalysator Chloride, Bromide und Jodide der Alkali- und Erdalkalielemente, der Elemente der Nebengruppe IIIb des Periodensystems, der Elemente mit der Ordnungszahl 24 bis 30, der Lanthanidenelemente, des Silbers, Cadmiums, Bleis und Wismuts einzeln oder im Gemisch einsetzt, wobei das Atomgewicht der Alkali-, Erdalkali- und IIIb-Nebengruppen-Elemente nicht größer als 139 ist.

2. Verfahren nach Anspruch 1, dadurch gekennzeichnet, daß man die Katalysatoren auf einem Träger einsetzt.

3. Verfahren nach den Ansprüchen 1 und 2, dadurch gekennzeichnet, daß man die Oxidation des Methans bei 650 bis 850 °C durchführt.

4. Verfahren nach den Ansprüchen 1 bis 3, dadurch gekennzeichnet, daß man gleichzeitig mit dem Methan und dem Sauerstoff oder dem sauerstoffhaltigen Gas den dem jeweils verwendeten Halogenid-Katalysator entsprechenden Halogenwasserstoff kontinuierlich oder intermittierend über den Katalysator leitet.

5. Verfahren nach Anspruch 4, dadurch gekennzeichnet, daß man als Halogenid-Katalysator Chloride verwendet und Chlorwasserstoff über den Katalysator leitet.

6. Verfahren nach Anspruch 1, dadurch gekennzeichnet, daß die Elemente der Ordnungszahl 24 bis 30 die Elemente Chrom, Mangan, Eisen, Kupfer und Zink sind.

7. Verfahren nach den Ansprüchen 2 bis 6, dadurch gekennzeichnet, daß man die Katalysatoren auf einem Träger einsetzt, welcher aus Bimsstein, Kieselgel, Kieselgur, gefällter Kieselsäure und/oder aus Oxiden der Erdalkalielemente und/oder Aluminiumoxid, Siliciumdioxid, Zinkoxid, Titandioxid, Zirkondioxid und/oder Siliciumcarbid besteht.

8. Verfahren nach Anspruch 7, dadurch gekennzeichnet, daß man als Träger Oxide der Erdalkalielemente Magnesium und/oder Calcium einsetzt.

9. Verfahren nach den Ansprüchen 1 bis 8, dadurch gekennzeichnet, daß 0,004 bis 0,300 Mol Katalysator auf 100 ml Träger eingesetzt werden.

10. Verfahren nach den Ansprüchen 1 bis 8, dadurch gekennzeichnet, daß 0,012 bis 0,190 Mol Katalysator auf 100 ml Träger eingesetzt werden.


**Claims**

1. A process for the production of ethylene and ethane by oxidation of methane with oxygen or an oxygen-containing gas in the presence of a metal compound as catalyst at a reaction temperature of from 600 to 1 000 °C, characterized in that chlorides, bromides and iodides of the alkali and alkaline earth elements, of elements of secondary group IIIb of the periodic system, of elements having ordinal numbers of from 24 to 30, of the lanthanide elements, of silver, cadmium, lead and bismuth are used either individually or in admixture as catalysts, the atomic weight of the alkali, alkaline earth and IIIb secondary group elements being no greater than 139.

2. A process as claimed in Claim 1, characterized in that the catalysts are applied to a support.

7

3. A process as claimed in Claims 1 and 2, characterized in that oxidation of the methane is carried out at 650 to 850 °C.

4. A process as claimed in Claims 1 to 3, characterized in that the hydrogen halide corresponding to the particular halide catalyst used is continuously or intermittently passed over the catalyst at the same time as the methane and the oxygen or the oxygen-containing gas.

5. A process as claimed in Claim 4, characterized in that chlorides are used as the halide catalyst and hydrogen chloride is passed over the catalyst.

6. A process as claimed in Claim 1, characterized in that the elements having ordinal numbers of from 24 to 30 are the elements chromium, manganese, iron, copper and zinc.

7. A process as claimed in Claims 2 to 6, characterized in that the catalysts are used on a support consisting of pumice, silica gel, kieselguhr, precipitated silica and/or of oxides of the alkaline earth elements and/or aluminium oxide, silicon dioxide, zinc oxide, titanium dioxide, zirconium dioxide and/or silicon carbide.

8. A process as claimed in Claim 7, characterized in that oxides of the alkaline earth elements magnesium and/or calcium are used as supports.

9. A process as claimed in Claims 1 to 8, characterized in that the catalyst is used in a quantity of from 0.004 to 0.300 mole to 100 ml of support.

10. A process as claimed in Claims 1 to 8, characterized in that the catalyst is used in a quantity of from 0.012 to 0.190 mole to 100 ml of support.

## Revendications

1. Procédé de préparation d'éthane et d'éthylène, par oxydation du méthane au moyen de l'oxygène ou d'un gaz contenant de l'oxygène, en présence d'un composé métallique utilisé comme catalyseur, à une température de réaction de 600 à 1 000 °C, caractérisé en ce que l'on utilise comme catalyseur les chlorures, bromures et iodures des éléments alcalins et alcalino-terreux, des éléments du sous-groupe IIIb du tableau périodique, des éléments de numéro atomique 24 à 30, des lanthanides, de l'argent, du cadmium, du plomb et du bismuth, seuls ou en mélange, la masse atomique des éléments alcalins et alcalino-terreux et de ceux du sous-groupe IIIb n'étant pas supérieure à 139.

2. Procédé conforme à la revendication 1, caractérisé en ce que l'on utilise les catalyseurs sur un support.

3. Procédé conforme aux revendications 1 et 2, caractérisé en ce que l'on effectue l'oxydation du méthane entre 650 et 850 °C.

4. Procédé conforme aux revendications 1 à 3, caractérisé en ce que l'on fait passer sur le catalyseur, en continu ou par intermittences, en même temps que le méthane et l'oxygène ou le gaz contenant de l'oxygène, l'halogénure d'hydrogène correspondant à l'halogénure utilisé comme catalyseur.

5. Procédé conforme à la revendication 4, caractérisé en ce que l'on utilise comme halogénure-catalyseur un chlorure, et que l'on fait passer sur le catalyseur du chlorure d'hydrogène.

6. Procédé conforme à la revendication 1, caractérisé en ce que les éléments de numéro atomique 24 à 30 sont le chrome, le manganèse, le fer, le cuivre et le zinc.

7. Procédé conforme aux revendications 2 à 6, caractérisé en ce que l'on utilise les catalyseurs sur un support, lequel support est constitué de pierre ponce, de gel de silice, de terre d'infusoires, d'acide silicique précipité, et/ou des oxydes des éléments alcalino-terreux et/ou d'oxyde d'aluminium, de dioxyde de silicium, d'oxyde de zinc, de dioxyde de titane, de dioxyde de zirconium et/ou de carbure de silicium.

8. Procédé conforme à la revendication 7, caractérisé en ce que l'on utilise comme support les oxydes des éléments alcalino-terreux magnésium et/ou calcium.

9. Procédé conforme aux revendications 1 à 8, caractérisé en ce que l'on utilise de 0,004 à 0,300 mole de catalyseur sur 100 ml de support.

10. Procédé conforme aux revendications 1 à 8, caractérisé en ce que l'on utilise de 0,012 à 0,190 mole de catalyseur sur 100 ml de support.